# EUROPEAN PATENT APPLICATION

(11) **EP 4 649 978 A2**
(43) Date of publication of application: **19.11.2025**
(21) Application number: 25205650.2
(22) Date of filing: 31.08.2021
(51) Int. Cl.: A61M 11/00

(54) **DRUG DELIVERY DEVICES WITH A MULTI-DISPENSING HEAD**

(30) Priority: 01.09.2020 US 202063073283 P
(62) Divisional of application: 21773004.3
(71) Applicant: Janssen Pharmaceutica NV, 2340 Beerse (BE)
(72) Inventor: SCRIMGEOUR, Ian, Dunbar, EH42 ILT (GB); HUBERT, Emma Louise, Milpitas, 95035 (US); GEARY, William C., Boston, 02111 (US); KAPIL, Monica A., San Jose, 95127 (US); RAMOS, David, Orange Park, 32065 (US)
(74) Representative: Carpmaels & Ransford LLP

(57) **Abstract**

Various exemplary drug delivery devices with a multi-dispensing head, drug products utilizing the same, and methods of using drug delivery devices with a multi-dispensing head are provided. In general, a nasal drug delivery device is configured to dispense a drug therefrom into a nose. In an exemplary embodiment, the drug delivery device includes two tips each configured to be positioned in or adjacent to a nostril. The two tips are configured to be positioned in or adjacent to the nostrils simultaneously.

## Description

### FIELD

The present disclosure relates generally to drug delivery devices with a multi-dispensing head and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration. However, these devices can require that the user reposition the device in each nostril for each delivery, which a user may not realize and/or may forget, such that the user only receives one spray and thus only a partial dose. These devices can be configured to only deliver one spray therefrom such that the user must use two devices to receive one dose of the drug, which a user may not realize and/or may forget, or the user may only have one device readily available, such that the user only receives one spray and thus only a partial dose.

Accordingly, there remains a need for improved nasal drug delivery devices.

### SUMMARY

**In** general, drug delivery devices with a multi-dispensing head, drug products utilizing the same, and methods of using drug delivery devices with a multi-dispensing head are provided.

**In** one aspect, a drug delivery system is provided that in one embodiment includes a first nasal drug delivery device, a second nasal drug delivery device, and a coupling mechanism. The first drug delivery devices includes a first tip configured to be positioned in a nose of a patient. The first tip has a first opening therein. The first drug delivery device also includes a first drug holder containing a first drug therein, and a first actuator configured to be actuated and thereby cause the first drug to be delivered out of the first opening. The second nasal drug delivery device includes a second tip configured to be positioned in the nose of the patient. The second tip has a second opening therein. The second drug delivery device also includes a second drug holder containing a second drug therein, and a second actuator configured to be actuated and thereby cause the second drug to be delivered out of the second opening. The coupling mechanism includes a body that defines first and second openings. The first opening is configured to releasably seat the first tip therein, and the second opening is configured to releasably seat the second tip therein.

The drug delivery system can have any number of variations. For example, the first and second openings can be located at respective proximal ends of the first and second drug delivery devices, and the coupling mechanism can be configured such that the first tip seated in the first opening faces proximally and the second tip seated in the second opening faces proximally. The first and second actuators can be configured to be selectively actuated so as to be actuated simultaneously to deliver the first and second drugs simultaneously into the nose or to be actuated sequentially to deliver the first and second drugs sequentially into the nose.

For another example, the first and second openings can be located at respective proximal ends of the first and second drug delivery devices, and the coupling mechanism can be configured such that the first tip seated in the first opening faces proximally and the second tip seated in the second opening faces distally. The first and second actuators can be configured to be actuated sequentially to deliver the first and second drugs sequentially into the nose.

For yet another example, the first and second openings can be located at respective proximal ends of the first and second drug delivery devices, and a proximal surface of the coupling mechanism can define a depth guide configured to contact a bottom surface of the nose with the coupling mechanism coupled to both of the first and second drug delivery devices and each of the first and second tips positioned in the nose. For still another example, the coupling mechanism can include a release mechanism configured to move between a closed state, in which the body defines an enclosed perimeter that defines an enclosed perimeter of each of the first and second openings, and an open state, in which the body has a discontinuous perimeter and neither of the first and second openings have an enclosed perimeter. For another example, the first and second openings can each have C-shapes that are back-to-back with one another. For still another example, the first and second drug can each be one of ketamine, esketamine, naloxone, and sumatriptan.

For another example, a drug delivery method can include actuating each of the first and second actuators and thereby causing the first drug to be delivered out of the first opening and causing the second drug to be delivered out of the second opening. The first and second drug can each be one of ketamine, esketamine, naloxone, and sumatriptan.

In another aspect, a drug product is provided that in one embodiment includes a drug product disposed in a drug delivery system. The drug product includes first and second drug products that each include one of ketamine, esketamine, naloxone, and sumatriptan. The drug delivery system includes a first nasal drug delivery device, a second nasal drug delivery device, and a coupling mechanism. The first drug delivery devices includes a first tip configured to be positioned in a nose of a patient. The first tip has a first opening therein. The first drug delivery device also includes a first drug holder containing the first drug product therein, and a first actuator configured to be actuated and thereby cause the first drug product to be delivered out of the first opening. The second nasal drug delivery device includes a second tip configured to be positioned in the nose of the patient. The second tip has a second opening therein. The second drug delivery device also includes a second drug holder containing the second drug product therein, and a second actuator configured to be actuated and thereby cause the second drug product to be delivered out of the second opening. The coupling mechanism includes a body that defines first and second openings. The first opening is configured to releasably seat the first tip therein, and the second opening is configured to releasably seat the second tip therein. The drug delivery system can have any number of variations.

In another aspect, a drug delivery device is provided that in one embodiment includes a first tip configured to be positioned in a first nostril of a patient. The first tip has a first opening therein. The drug delivery device also includes a first drug holder containing a first drug therein configured to be delivered out of the first opening, and a second tip configured to be positioned in a second nostril of the patient such that the first tip is positioned in the first nostril and the second tip is positioned in the second nostril. The second tip has a second opening therein. The drug delivery device also includes a second drug holder containing a second drug therein configured to be delivered out of the second opening, and a viewing window configured to indicate whether the first drug has or has not been delivered out of the first opening and whether the second drug has or has not bene delivered out of the second opening.

The drug delivery device can have any number of variations. For example, the drug delivery device can also include a first actuator configured to be actuated and thereby cause the first drug to be delivered out of the first opening, the drug delivery device can also include a second actuator configured to be actuated and thereby cause the second drug to be delivered out of the second opening, the actuation of the first actuator can automatically cause the viewing window to indicate that the first drug has been delivered, and the actuation of the second actuator can automatically cause the viewing window to indicate that the second drug has been delivered. The drug delivery device can also include a first spring configured to be released in response to the actuation of the first actuator, the release of the first spring can provide a force that moves the first drug holder relative to the first tip and thereby causes delivery of the first drug, the drug delivery device can also include a second spring configured to be released in response to the actuation of the second actuator, and the release of the second spring can provide a force that moves the second drug holder relative to the second tip and thereby causes delivery of the second drug.

For another example, the drug delivery device can also include a single actuator configured to be actuated and thereby cause the first and second drugs to be delivered out of the first and second openings, respectively, and the actuation of the actuator can automatically cause the viewing window to indicate that the first and second drugs have been delivered. The drug delivery device can also include a locking mechanism configured to automatically lock the actuator in position relative to the first and second tips in response to the actuation of the actuator. The drug delivery device can also include a spring configured to be released in response to the actuation of the actuator, and the release of the spring can provide a force that moves the first and second drug holders relative to the first and second tips and thereby causes delivery of the first and second drugs.

For yet another example, a dispensing head of the drug delivery device can have the first and second tips integrally formed therewith. For another example, the first drug holder can include a first portion that is a first color and a second portion that is a second color, the second drug holder can include a first portion that is the first color and a second portion that is the second color, the first color being visible in the viewing window can be indicative that drug delivery has not occurred, and the second color being visible in the viewing window can be indicative that drug delivery has occurred. For still another example, the first and second drugs can each be one of ketamine, esketamine, naloxone, and sumatriptan.

For another example, a drug delivery method can include causing each of the first and second drugs to be delivered out of the first and second openings, respectively. The first and second drugs can each be one of ketamine, esketamine, naloxone, and sumatriptan.

In another aspect, a drug product is provided that in one embodiment includes a drug product disposed in a drug delivery device. The drug product includes first and second drug products that each include one of ketamine, esketamine, naloxone, and sumatriptan. The drug delivery device includes a first tip configured to be positioned in a first nostril of a patient. The first tip has a first opening therein. The drug delivery device also includes a first drug holder containing the first drug t product herein configured to be delivered out of the first opening, and a second tip configured to be positioned in a second nostril of the patient such that the first tip is positioned in the first nostril and the second tip is positioned in the second nostril. The second tip has a second opening therein. The drug delivery device also includes a second drug holder containing the second drug product therein configured to be delivered out of the second opening, and a viewing window configured to indicate whether the first drug product has or has not been delivered out of the first opening and whether the second drug product has or has not bene delivered out of the second opening. The drug delivery device can have any number of variations.

### BRIEF DESCRIPTION OF DRAWINGS

The present invention is described by way of reference to the accompanying figures which are as follows:
FIG. 1 is a block diagram of one embodiment of a drug delivery device;
FIG. 2 is a side view of one embodiment of the drug delivery device of FIG. 1;
FIG. 3 is a side view of another embodiment of the drug delivery device of FIG. 1, the drug delivery device being in a pre use state;
FIG. 4 is a side view of the drug delivery device of FIG. 3 in a post use state;
FIG. 5 is a side cross-sectional view of yet another embodiment of the drug delivery device of FIG. 1;
FIG. 6 is a perspective view of one embodiment of a coupling mechanism configured to couple together two drug delivery devices;
FIG. 6A is a front view of a nose of a user;
FIG. 6B is a front view of a nose of another user;
FIG. 7 is a perspective view of the coupling mechanism of FIG. 6 coupling two drug delivery devices together;
FIG. 8 is a perspective view of another embodiment of a coupling mechanism coupling two drug delivery devices of FIG. 2 together;
FIG. 9 is a perspective view of another embodiment of a coupling mechanism and two drug delivery devices of FIG. 2 configured to be coupled together by the coupling mechanism;
FIG. 10 is a side view of another embodiment of a coupling mechanism configured to couple together two drug delivery devices;
FIG. 11 is a side view of the coupling mechanism of FIG. 6 coupling two drug delivery devices of FIG. 2 together;
FIG. 12 is a perspective view of yet another embodiment of a coupling mechanism;
FIG. 13 is a side view of the drug delivery device of FIG. 2 being coupled to the coupling mechanism of FIG. 12;
FIG. 14 is a perspective view of another embodiment of the drug delivery device of FIG. 1;
FIG. 15 is a side view of the drug delivery device of FIG. 14;
FIG. 16 is a side view of the drug delivery device of FIG. 14 after delivery of one drug spray therefrom;
FIG. 17 is a side view of the drug delivery device of FIG. 15 after delivery of a second drug spray therefrom;
FIG. 18 is a side view of yet another embodiment of the drug delivery device of FIG. 1;
FIG. 19 is a side view of the drug delivery device of FIG. 18 after delivery of one drug spray therefrom;
FIG. 20 is a side view of the drug delivery device of FIG. 19 after delivery of a second drug spray therefrom;
FIG. 21 is a perspective view of still another embodiment of the drug delivery device of FIG. 1;
FIG. 22 is a side view of the drug delivery device of FIG. 21;
FIG. 23 is a side view of the drug delivery device of FIG. 21 after delivery of two drug sprays therefrom;
FIG. 24 is a side view of another embodiment of the drug delivery device of FIG. 1;
FIG. 25 is a perspective view of a portion of the drug delivery device of FIG. 24;
FIG. 26 is a side view of still another embodiment of the drug delivery device of FIG. 1;
FIG. 27 is a side view of yet another embodiment of the drug delivery device of FIG. 1; and
FIG. 28 is a side cross-sectional view of another embodiment of the drug delivery device of FIG. 1.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with a multi-dispensing head, drug products utilizing the same, and methods of using drug delivery devices with a multi-dispensing head are provided. In general, a nasal drug delivery device is configured to dispense a drug therefrom into a nose. In an exemplary embodiment, the drug delivery device includes two tips each configured to be positioned in or adjacent to a nostril. The two tips are configured to be positioned in or adjacent to the nostrils simultaneously, which may help ensure that the tips are positioned correctly relative to the nose for drug delivery into each nostril since a user of the drug delivery device will be able to see and feel not just one tip but two tips. The two tips being configured to be positioned in or adjacent to the nostrils simultaneously may allow for the same drug delivery device to deliver multiple drug sprays to a user, e.g., one spray from each tip, which may reduce medical waste compared to drug delivery devices with only one tip configured to deliver only one drug spray therefrom since the drug delivery device is usable for multiple drug sprays.

Some drug doses may require two drug sprays, e.g., one spray into each nostril. For such drug doses, the drug delivery device including two tips being configured to be positioned in or adjacent to the nostrils simultaneously may help ensure that a user of the drug delivery device receives both of the drug sprays since the drug sprays may be deliverable simultaneously from the two tips, and/or the two tips may provide a visual clue to the user that a drug spray is needed in each nostril such that the user is less likely to deliver only a partial dose, e.g., one drug spray, from the drug delivery device in embodiments in which the drug delivery device is configured to be separately actuated for each drug spray therefrom.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient can have a variety of configurations. In general, the drug delivery device is configured to deliver a drug to a patient, where the drug is provided in a defined dosage form within the drug delivery device.

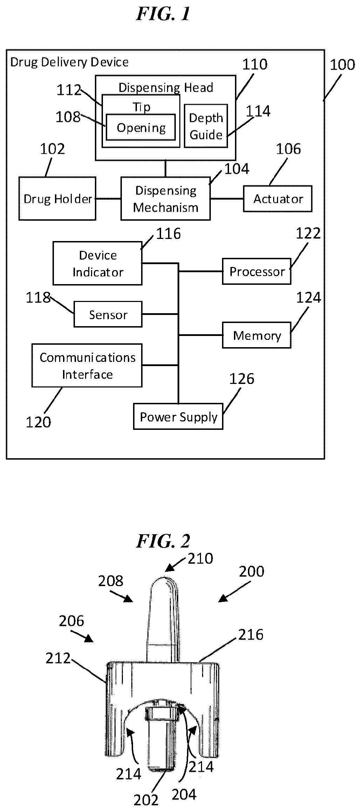

FIG. 1 illustrates one embodiment of a drug delivery device 100 configured to expel a drug into a nose of a patient. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, such as a cartridge, a vial, a blow-fill-seal (BFS) capsule, a blister pack, etc.

The drug delivery device 100 does not need to be primed after the drug holder 102 has been coupled thereto and prior to actuation of the drug delivery device 100 to cause drug delivery, which may ease use of the drug delivery device 100 by a user.

The drug delivery device 100 also includes a dispensing mechanism 104 that is operatively coupled to the drug holder 102 and configured to drive the drug out of device 100 from the drug holder 102. The dispensing mechanism 104 can have a variety of configurations. For example, the dispensing mechanism 104 can include a plunger configured to push the drug out of the drug holder 102. For another example, the dispensing mechanism 104 can include a piston, pin, and/or a needle configured to pierce through or puncture a seal member of the drug holder 102 in embodiments in which the drug holder 102 includes a pierceable or puncturable seal member.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a plunger. For another example, the actuator 106 can include a pressable button. For still another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100 in a tip 112 of the dispensing head 110. The tip 112 is configured to be inserted into or be positioned adjacent to a nostril of a patient. In some embodiments, the tip 112 includes a single tip configured to be inserted into or positioned adjacent to a first nostril of the patient during a first stage of operation of the drug delivery device 100 and into or adjacent to a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. In some embodiments, the tip 112 includes two tips each configured to be inserted into or positioned adjacent to one nostril of a patient such that both tips 112 are inserted into nostrils at the same time. The actuator 106 is configured to be actuated to simultaneously cause a first dose of the drug to be delivered through one tip 112 and a second dose of the drug to be delivered through the other tip 112.

The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112. A person skilled in the art will appreciate that the longitudinal axes may not be precisely aligned but nevertheless be considered to be substantially aligned due to any number of factors, such as manufacturing tolerances and sensitivity of measurement equipment. In embodiments including two tips 112, a single depth guide 114 can be used for both tips 112, or each of the tips 112 can have a dedicated depth guide 114.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 110, and/or and to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

FIG. 2 illustrates an embodiment of the drug delivery device 100 of FIG. 1. The drug delivery device 200 of FIG. 2 is generally configured and used similar to that discussed above regarding the drug delivery device 100 of FIG. 1 and includes a drug holder 202, a piston 204, and a dispensing head 206 including a tip 208 and an opening 210. The dispensing head 206 has the piston 204 fixed thereto. From an inner end face of the piston 204 emanates an outlet channel, which is guided in valve-free manner to the opening 210 leading into the open and which is formed by an atomizing nozzle. The outlet channel becomes to be in fluid communication with a hollow interior of the drug holder 202 in response to the piston 204 puncturing or piercing a proximal seal member of the drug holder 202 as the drug holder 202 is pushed proximally, e.g., in a direction toward the opening 210. The dispensing head 206 includes a handle portion 212 configured to be handheld. Flattened sides of the handle portion 212 include two facing finger openings 214 configured to receive a thumb of a user's hand with the index and middle finger of this hand on either side of a discharge connection of the handle portion 212 on an outside of the handle portion's end wall 216 or elsewhere on the handle portion 212. By moving together the thumb and the two other fingers the drug holder 202 can be displaced with respect to the piston 204 or the dispensing head 206 and the drug consequently sprayed out of the drug holder 202 through a discharge channel of the drug delivery device 200 and through the opening 210. The drug delivery device 200 is further described in U.S. Pat. No. 4,946,069 entitled "Dispenser For Manually Discharging Flowable Media" issued Aug. 7, 1990, which is hereby incorporated by reference in its entirety.

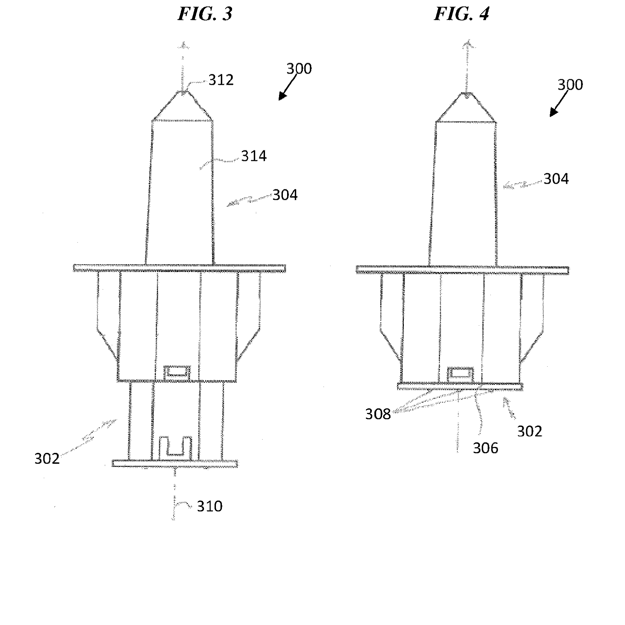

FIGS. 3 and 4 illustrate another embodiment of the drug delivery device 100 of FIG. 1. The drug delivery device 300 of FIGS. 3 and 4 is generally configured and used similar to that discussed above regarding the drug delivery device of FIG. 1. FIG. 3 shows this illustrated embodiment of a drug delivery device 300 in an initial or pre-use configuration, and FIG. 4 shows the drug delivery device 300 in an actuated or post use configuration. In the post use configuration, a body 302 of the drug delivery device 300 has been inserted into a dispensing head 304 of the drug delivery device 300 such that only an endplate 306 of the body 302 is visible.

The body 302, which includes a drug holder therein, is configured to be inserted into the dispensing head 304 substantially along a first axis 310. A person skilled in the art will appreciate that the body 302 may not be inserted precisely along the first axis 310 but nevertheless be considered to be inserted substantially along the first axis 310 due to any number of factors, such as manufacturing tolerances and sensitivity of measurement equipment. The body 302 defines an actuator of the drug delivery device 300 that is configured to be manually moved by a user to be inserted into the dispensing head 304 and cause drug delivery. The endplate 306 includes a gripping feature 308 configured to facilitate actuation by increasing grip of the body's surface for a finger of a user. The gripping feature 308 includes surface projections in this illustrated embodiment but can have other configurations, such as a textured surface, a finger depression, etc.

During use of the drug delivery device 300, an insertion force is applied to the body 302 such that the body 302 slides proximally, e.g., toward an opening 312 in a tip 314 of the dispensing head 304, relative to the dispensing head 304. As the body 302 is moved in the proximal direction, a piercing element punctures or pierces a proximal seal member of the drug holder. Further movement of the body 302 in the proximal direction causes a piercer stop of a holder holding the piercing element in the dispensing head 304 to come into contact with the drug holder. The contact between the piercer stop and the drug holder is configured to prevent further insertion of the body 302 in the proximal direction, which can act as an indicator to a user of the drug delivery device 300 that actuation has been completed.

The drug delivery device 300 is further described in U.S. Pat. Pub. No. 2018/0361085 entitled "Nasal Spray Assembly" published Dec. 20, 2018, which is hereby incorporated by reference in its entirety.

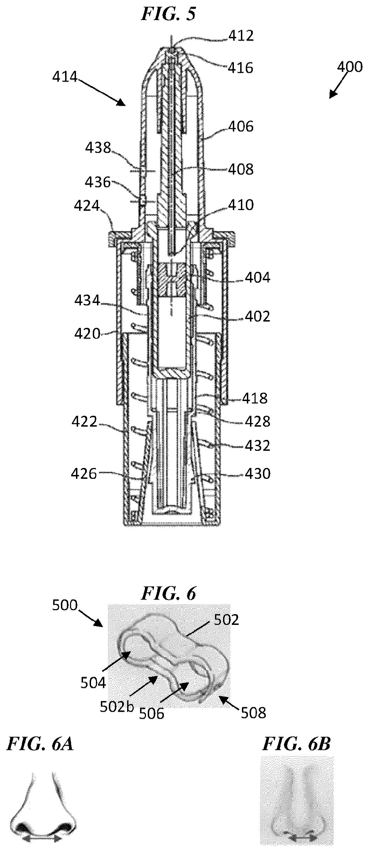

FIG. 5 illustrates yet another embodiment of the drug delivery device 100 of FIG. 1. The drug delivery device 400 is generally configured and used similar to that discussed above regarding the drug delivery device 100 of FIG. 1 and includes a drug holder 402 containing two doses of drug therein. A dispensing mechanism, such as a piston 404, is mounted to slide in the drug holder 402. In a pre-actuation position of the drug delivery device 400, shown in FIG. 4, the piston 404 acts as a stopper, isolating the drug. The drug delivery device 400 also includes a dispensing head 406 that is assembled on the drug holder 402 and that is configured to be axially movable relative to the drug holder 402. In particular, an axial movement of the dispensing head 406 relative to the drug holder 402 causes the piston 404 to move in the drug holder 402, and thus causes the drug contained in the drug holder 402 to be dispensed. The dispensing head 406 includes a dispenser channel 408 that extends from a perforator tip 410 to an opening 412 in a tip 414 of the dispensing head 406. Proximal to or upstream of the opening 412, a spray profile 416 can be provided that is configured to dispense the drug in the form of spray.

The drug holder 402 is fastened in a body 418 that is thus secured to the drug holder 402 and that moves together with the drug holder 402.

The dispensing head 406 includes a bottom side skirt 420 that is adapted to cooperate with an actuator 422 of the drug delivery device 400. A finger-rest element 424 can be assembled around the dispensing head 406 or can be formed integrally therewith.

The actuator 422 is configured to be axially movable inside the side skirt 420 of the dispensing head 406 so as to perform successive actuations of the drug delivery device 400. The actuator 422 includes at least one sloping tab 426 that is configured to cooperate with projections 428, 430 of the body 418 so as to perform successive actuations. A return spring 432 is mounted between the actuator 422 and the dispensing head 406 so as to return the actuator 422 into its start position after each actuation.

The drug delivery device 400 includes a device indicator 434 configured to indicate to a user whether a first dose has been dispensed and whether a second dose has been dispensed. In this way, the user knows exactly what the situation is, and whether or not the first dose has been dispensed. The indicator 434 is configured to cooperate with viewing windows 436, 438 that are formed in the dispensing head 406. The viewing windows 436, 438 are formed in a side wall of the dispensing head 406, clearly visible to the user when the drug delivery device 400 is held in the hand.

The drug delivery device 400 is further described in U.S. Pat. No. 9,555,950 entitled "Fluid Product Dispensing Device" issued Jan. 31, 2017, which is hereby incorporated by reference in its entirety.

Various other embodiments of the drug delivery device 100 are further discussed in U.S. Pat. No. 7,299,949 entitled "Discharge Device For Manually Producing A Volume Flow" issued Nov. 27, 2007, U.S. Pat. No. 6,321,942 entitled "Discharge Device For Flowable Media Using A Thrust Piston Pump" issued Nov. 27, 2001, U.S. Pat. No. 9,314,808 entitled "Fluid Delivery Device" issued Apr. 19, 2016, U.S. Pat. Pub. No. 2015/0274344 entitled "Devices And Methods For Packaging And Dispensing Unit Doses Of Personal Care Products" published Oct. 1, 2015, U.S. Pat. No. 8,585,659 entitled "Piercing Device For Drug Delivery Systems" issued Nov. 19, 2013, U.S. Pat. Pub. No. 2015/0297845 entitled "Nasal Delivery Device" published Oct. 22, 2015, and U.S. Pat. Pub. No. 2018/0060527 entitled "System And Method For Controlled Medical Therapy" published Mar. 1, 2018, which are hereby incorporated by reference in their entireties. Nasal spray syringes are also examples of the drug delivery device 100.

The drug delivery device 100 of FIG. 1, including the various embodiments thereof discussed herein, can be configured to be releasably coupled to a second drug delivery device 100 to form a combination drug delivery device. Each of the two drug delivery devices 100 can include a single tip such that the resulting combination drug delivery device includes two tips each configured to deliver a drug spray through an opening formed in the tip. The combination drug delivery device may help ensure that the tips are, at the same time, positioned correctly relative to the nose for drug delivery into each of two nostrils since a user of the combination drug delivery device will be able to see and feel not just one tip but two tips, and/or may help ensure that a user has two drug sprays available to achieve a full dose that requires two drug sprays. The two drug delivery devices being coupled together may allow existing drug delivery devices to be used in forming a combination drug delivery device such that the drug delivery device need not be redesigned and the current manufacturing technique of the drug delivery device can be maintained.

The two drug delivery devices can be coupled together in a variety of ways. In general, a coupling mechanism can be configured to releasably coupled two drug delivery devices together. In embodiments in which the drug delivery devices coupled together using the coupling mechanism include a device indicator that would be fully or partially obscured by the coupling mechanism coupled to the drug delivery devices, the coupling mechanism can be transparent in at least a portion thereof to allow visualization of the device indicator through the transparent area or can include hole(s) in the body thereof to allow visualization of the device indicator therethrough and/or facilitate sound travel therethrough.

The coupling mechanisms described herein can be made from any of a variety of materials. Examples of materials that can be used include metal (stainless steel, titanium, etc.), a plastic or other polymer, and rubber. In an exemplary embodiment, the coupling mechanism is made from one or more nontoxic and/or biocompatible materials.

FIG. 6 illustrates one embodiment of a coupling mechanism configured to releasably couple two drug delivery devices together. The coupling mechanism in this illustrated embodiment includes a clip 500 configured to clip two of the drug delivery devices 100 together. The clip 500 includes a body 502 that defines two cylindrical passages 504, 506. Each of the passages 504, 506 is configured to securely seat one of the drug delivery devices 100 therein. Each of the passages 504, 506 has a circumference just slightly larger than an outer circumference of a portion of the drug delivery device 100 to be seated therein to allow an interior surface of the body 502 that defines the passages 504, 506 to securely grip the drug delivery device 100. Alternatively, each of the passages 504, 506 can have a circumference that is less than or equal to the outer circumference of the body 502 of the drug delivery device 100, and at least a portion of the interior surface of the body 502 that defines the passages 504, 506 can be formed of or have thereon a plastic or resilient material, e.g., rubber, etc., configured to securely grip the drug delivery device 100, e.g., in an interference fit. The portion of the drug delivery device 100 to be seated in one of the passages 504, 506 can include the dispensing head 110, e.g., the tip 112 thereof, or another portion of the drug delivery device 100, such as a body of the drug delivery device 100 that is attached to the dispensing head 110 and that houses various components of the drug delivery device 100 therein. The interior surface of the body 502 of the clip 500 that defines the passages 504, 506 can include a gripping feature configured to facilitate secure seating of the drug delivery device 100 therein. The gripping feature can have any of a variety of configurations, such as a textured surface, a rubber lining on the interior surface of the body 502, etc.

The clip 500 in this illustrated embodiment includes a release mechanism 508 configured to facilitate coupling of the two drug delivery devices 100 to the clip 500 and decoupling of the two drug delivery devices 100 from the clip 500. Decoupling the drug delivery devices 100 from the clip 500 allows the clip 500 to be reused with other drug delivery devices 100 same or different from the first two drug delivery devices 100 attached together using the clip 500. The release mechanism 508 is configured to move between a closed state, in which the clip's body 502 defines an enclosed perimeter, and an open state, in which the clip's body 502 has a discontinuous perimeter. The release mechanism 508 is shown in the closed state in FIG. 6. The release mechanism 508 can have a variety of configurations. For example, as shown in this illustrated embodiment, the release mechanism 508 can include a hole formed through one end of the body 502 and a protrusion extending from the other end of the body 502 that is configured to releasably seat in the hole. For another example, the release mechanism 508 can include Velcro^{®} on each end of the body 502. For yet another example, the release mechanism 508 can include a magnet on each end of the body 502. For still another example, the release mechanism 508 can include a snap connection.

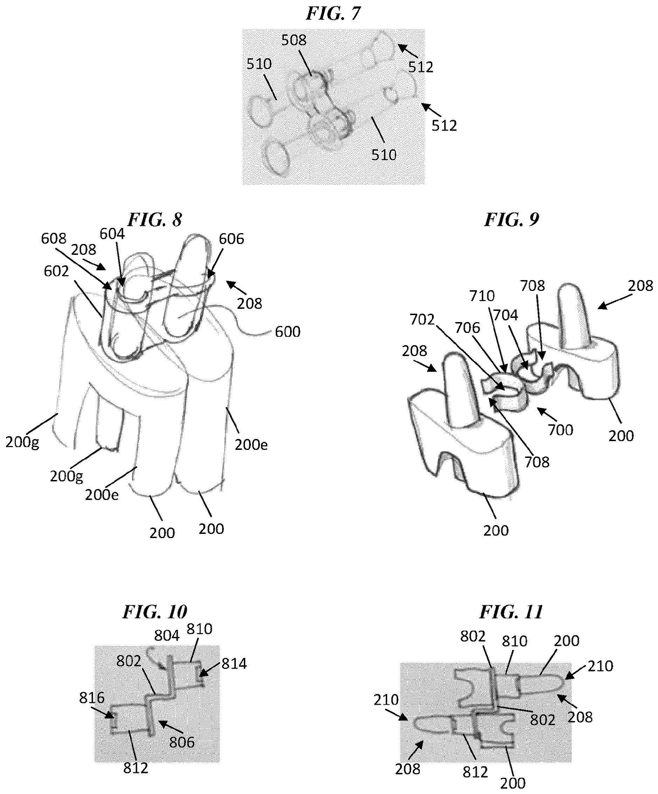

FIG. 7 illustrates two drug delivery devices 510, each including a tip 512, releasably coupled together with the clip 500. Each of the drug delivery devices 510 is another embodiment of the drug delivery device 100 of FIG. 1 and is generally configured and used similar to that discussed above regarding the drug delivery device 100 of FIG. 1. The drug delivery device's tip 512 in this illustrated embodiment is not configured to be disposed in a nostril but instead to be positioned adjacent to a nostril by abutting a bottom surface of a nose under the nostril to deliver drug into the nostril.

In the illustrated embodiment of FIGS. 6 and 7, the passages 504, 506 of the clip 500 are at a fixed distance from one another. In other embodiments, the clip 500 can be configured to allow a distance between the passages 504, 506 to vary from one another. In other words, a spacing between the passages 504, 506 can be variable. Having a variable distance between the passages 504, 506 may help accommodate different anatomies (e.g., different size noses, different size nostrils, or a child's nose compared to an adult's nose) by helping tips of drug delivery devices positioned in each of the passages 504, 506 to be comfortably positioned in a particular user's nostrils by adjusting the distance between the passages 504, 506. For example, FIG. 6A illustrates a first distance D1 between two nostrils of a first patient, and FIG. 6B illustrates a second distance D2 between two nostrils of a second patient. The second distance D2 is less than the first distance D2 such that a smaller distance between the passages 504, 506 for the second patient, as compared to the first patient, may result in a more comfortable drug delivery experience.

A bridge portion 502b of the clip's body 500 can be adjustable in length so as to allow for variable distances between the passages 504, 506. The distance between the passages 504, 506 can be configured to be varied in a variety of ways. For example, each of the two opposed sides of the clip 500 in the bridge portion 502b could be accordion folded and configured to be selectively unfolded at each of the folds to allow for more or less folds depending on a desired distance between the passages 504, 506. For another example, the first passage 504 can be formed in a first portion of the clip 500 that includes a first portion of the bridge portion 502b, and the second passage 506 can be formed in a second portion of the clip 500 that includes a second portion of the bridge portion 502b. One of the first and second portions can be telescoped in the other of the first and second portions such that by sliding one of the first and second portions relative to the other, the distance between the passages 504, 506 can be changed. In some embodiments, the first and second portions are configured to be freely slidable relative to one another such that any distance between the passages 504, 506 can be selected. In other embodiments, the first and second portions can include a mechanism configured to allow the first and second portions to be positioned relative to one another at any of a plurality of predetermined positions, such as by using a tooth/rack mechanism or other ratchet mechanism.

FIG. 8 illustrates another embodiment of a coupling mechanism 600 configured to releasably couple two drug delivery devices together. The drug delivery devices shown in FIG. 8 are each the drug delivery device 200 of FIG. 2 but other embodiments of drug delivery devices can be similarly coupled together using the coupling mechanism 600. The coupling mechanism 600 of FIG. 8 is generally configured and used similar to the coupling mechanism 500 of FIG. 7 except that the coupling mechanism 600 does not include a release mechanism.

The coupling mechanism 600 includes a body 602 that defines two cylindrical passages 604, 606. Each of the passages 604, 606 is configured to securely seat one of the drug delivery devices 200 therein by seating the tip 208 therein as shown in FIG. 8. Each of the passages 604, 606 has a circumference just slightly larger than an outer circumference of a portion of the drug delivery device 200 to be seated therein, to allow an interior surface of the body 602 that defines the passages 604, 606 to securely grip the drug delivery device 200. The passages 604, 606 seat the tips 208 in this illustrated embodiment but can seat other portions of the drug delivery devices 200, such as first legs 200e on one side thereof, second legs 200g on another side thereof, etc. The interior surface of the body 602 of the clip 600 that defines the passages 604, 606 can include a gripping feature configured to facilitate secure seating of the drug delivery device 200 therein. The gripping feature can have any of a variety of configurations, such as a textured surface, a rubber lining on the interior surface of the body 602, etc.

A proximal surface 608 of the coupling mechanism 600 defines a depth guide for the drug delivery devices 200 with the coupling mechanism 600 coupled to the drug delivery devices 200.

The coupling mechanism 600 is configured to be releasably coupled to the drug delivery devices 200 by being slid over each of the tips 208. Either the coupling mechanism 600 can be held in a fixed position while the drug delivery devices 200 are slid through the passages 604, 606, or the drug delivery devices 200 can be held in a fixed position while the coupling mechanism 600 is slid over the tips 208. The drug delivery device 200 can be simultaneously coupled to the coupling mechanism 600 or can be sequentially coupled to the coupling mechanism 600. The coupling mechanism 600 is configured to be released from the drug delivery devices 600 by the coupling mechanism 600 being slid off the tips 208 while the drug delivery devices 200 are held in a fixed position or by the tips 208 being slid out of the passages 604, 606 while the coupling mechanism 600 is held in a fixed position.

FIG. 9 illustrates another embodiment of a coupling mechanism 700 configured to releasably couple two drug delivery devices together. The drug delivery devices shown in FIG. 9 are each the drug delivery device 200 of FIG. 2 but other embodiments of drug delivery devices can be similarly coupled together using the coupling mechanism 700. The coupling mechanism 700 of FIG. 9 is generally configured and used similar to the coupling mechanism 600 of FIG. 8 except that passages 702, 704 of the coupling mechanism 700 are not enclosed passages defined by the coupling mechanism's body 706. Instead, the passages 702, 704 are each open with the body 706 including opposed C-shaped members that define the passages 702, 704.

The coupling mechanism 700 is configured to be releasably coupled to the drug delivery devices 200, such as by being slid over a side each of the tips 208 or other portions of the drug delivery devices 200, such as first legs 200e on one side thereof, second legs 200g on another side thereof, etc. Either the coupling mechanism 700 can be held in a fixed position while the drug delivery devices 700 are slid through a side opening 708 of each of the passages 702, 704, or the drug delivery devices 200 can be held in a fixed position while the coupling mechanism 700 is slid onto the tips 208. The drug delivery device 200 can be simultaneously coupled to the coupling mechanism 700 or can be sequentially coupled to the coupling mechanism 700. The coupling mechanism 700 is configured to be released from the drug delivery devices 700 by the coupling mechanism 700 being slid off the tips 208 while the drug delivery devices 200 are held in a fixed position or by the tips 708 being slid out of the passages 702, 704 while the coupling mechanism 700 is held in a fixed position.

Free ends of each C-shaped portion of the body 706 can be biased radially inward and can be configured to dynamically flex radially outward. The free ends can thus be configured to flex radially outward during coupling of the drug delivery devices 200 to the coupling mechanism 700 and be biased radially inward toward the tips 208 seated in the passages 702, 704, which may facilitate seating of the tips 208 in the passages 702, 704 and/or the coupling mechanism's gripping of the tips 208 seated in the passages 702, 704. The coupling mechanism 700 can similarly dynamically flex radially outward during decoupling of the drug delivery devices 200 from the coupling mechanism 700.

A proximal surface 710 of the coupling mechanism 700 defines a depth guide for the drug delivery devices 200 with the coupling mechanism 700 coupled to the drug delivery devices 200.

The coupling mechanisms 500, 600, 700 of FIGS. 6-9 are configured to allow the openings in the tips of the drug delivery devices coupled thereto to each face a same direction, as shown in FIGS. 7-9. The two tips can thus be simultaneously positioned in two nostrils. The tips being simultaneously positioned in two nostrils may allow the drug delivery devices to be actuated at substantially the same time as one another, which may improve user experience by allowing for faster dosing than sequentially delivered doses. A person skilled in the art will appreciate that the actuations may not occur at precisely the same time as one another but nevertheless be considered to be at substantially the same time as one another. The coupling mechanisms 600, 700 of FIGS. 6-9 are also configured to allow the openings in the tips of the drug delivery devices coupled thereto to each face a different direction. The two tips thus cannot be simultaneously positioned in two nostrils but can be sequentially positioned in two nostrils. The tips being sequentially positioned in two nostrils requires that each of the drug delivery devices coupled together be separately actuated, which may help ensure that each of the drug delivery devices is fully and properly actuated.

FIGS. 10 and 11 illustrate another embodiment of a coupling mechanism 800 configured to releasably couple two drug delivery devices together. The drug delivery devices shown in FIG. 11 are each the drug delivery device 200 of FIG. 2 but other embodiments of drug delivery devices can be similarly coupled together using the coupling mechanism 800. The coupling mechanism 800 of FIGS. 10 and 11 is generally configured and used similar to the coupling mechanism 600 of FIG. 8 except that the coupling mechanism 800 is configured to only allow the openings 210 in the tips 208 of the drug delivery devices 200 coupled thereto to each face a different direction, e.g., the openings 210 in the tips 208 of the drug delivery devices 200 coupled thereto cannot face the direction.

The coupling mechanism 800 includes a body 802 that defines two cylindrical passages 804, 806. Each of the passages 804, 806 is configured to securely seat one of the drug delivery devices 200 therein by seating the tip 208 therein, as shown in FIG. 11. Each of the passages 804, 806 has a circumference just slightly larger than an outer circumference of a portion of the drug delivery device 200 to be seated therein, e.g., the tip 208, to allow an interior surface of the body 802 that defines the passages 804, 806 to securely grip the drug delivery device 200. The body 802 includes first and second extensions 810, 812 that extend from a main portion of the body 802 in opposite directions. The extensions 810, 812 are each configured to seat a tip 208 therein, as shown in FIG. 11. The extensions 810, 812 act as a guide for the drug delivery devices 200 to be coupled thereto by indicating where the tips 208 should be positioned relative to the coupling mechanism 800. The extensions 810, 812 facilitate the coupling of the drug delivery devices 200 together with their tips 208 facing in opposite directions. The extensions 810, 812 can each be tapered in a direction away from the main portion of the body 802, which may further help guide the tips 208 in position relative to each other and to the coupling mechanism 800.

The interior surface of the body 802 of the coupling mechanism 800 that defines the passages 804, 806, e.g., the interior surfaces of the extensions 810, 812, can include a gripping feature configured to facilitate secure seating of the drug delivery device 200 therein. The gripping feature can have any of a variety of configurations, such as a textured surface, a rubber lining on the interior surface of the body 802, etc.

A proximal surface of each extension 810, 812 defines a depth guide for the drug delivery device 200 coupled thereto.

The coupling mechanism 800 in this illustrated embodiments includes a pair of cut-outs 814, 816 therein. The cut-outs 814, 816 are in the extensions 810, 812 that receive the tips 802 therein. The cut-outs 814, 816 are configured to provide a window for a device indicator on a tip of the drug delivery device seated in its associated extension 810, 812 so the device indicator will not be obscured by the extension 810, 812 but instead be positioned for viewing in the cut-out 814, 816.

The coupling mechanism 800 is configured to be releasably coupled to the drug delivery devices 200 by being slid over each of the tips 208 similar to that discussed above regarding the coupling mechanism 600 of FIG. 8 and to be decoupled from the drug delivery devices 200 similar to that discussed above regarding the coupling mechanism 600 of FIG. 8.

The coupling mechanisms 500, 600, 700, 800 of FIGS. 6-11 are configured to simultaneously couple to two drug delivery devices. **In** other embodiments, a coupling mechanism is configured to sequentially couple to two different drug delivery devices or to the same drug delivery device. In such embodiments, the coupling mechanism is configured to facilitate proper positioning of the drug delivery devices in two nostrils. Also, as mentioned above, tips being sequentially positioned in two nostrils requires that each of the drug delivery devices coupled together be separately actuated, which may help ensure that each of the drug delivery devices is fully and properly actuated.

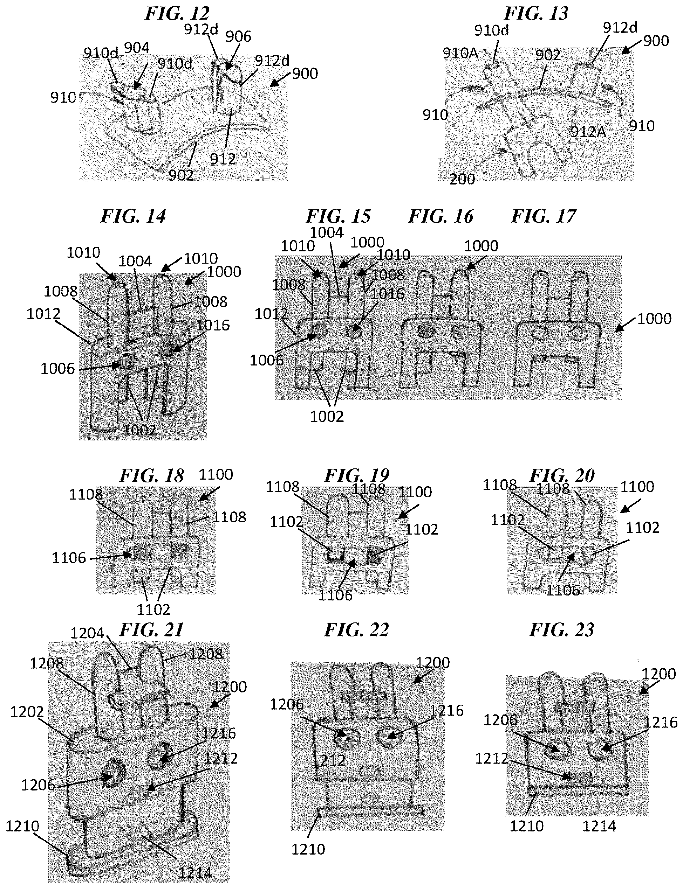

FIGS. 12 and 13 illustrate another embodiment of a coupling mechanism 900. The coupling mechanism 900 of FIGS. 12 and 13 is configured to sequentially couple to two different drug delivery devices or to the same drug delivery device, to the drug delivery device 200 of FIG. 13 twice, to the drug delivery device 200 of FIG. 13 once and then to another drug delivery device (or vice versa), to another drug delivery device twice, or to two other different drug delivery devices. The drug delivery device shown in FIG. 13 is the drug delivery device 200 of FIG. 2 but other embodiments of drug delivery devices can be similarly coupled using the coupling mechanism 900.

The coupling mechanism 900 includes a body 902 that includes first and second extensions 910, 912 each extending from a main portion of the body 802 in a same direction as one another. The body 902, e.g., the extensions 910, 912 thereof, defines two cylindrical passages 904, 906. Each of the passages 904, 906 is configured to securely seat a drug delivery device therein by seating the drug delivery device's tip therein. Each of the passages 904, 906 has a circumference just slightly larger than an outer circumference of a portion of the drug delivery device to be seated therein, e.g., the tip thereof, to allow an interior surface of the body 902 that defines the passages 904, 906 to securely grip the drug delivery device 200. The body 902 includes first and second extensions 910, 912 that extend from a main portion of the body 902 in opposite directions. The extensions 910, 912 are each configured to seat a drug delivery device's tip therein. The extensions 910, 912 act as a guide for the drug delivery devices to be coupled thereto by indicating where the tips should be positioned relative to the coupling mechanism 900. The extensions 910, 912 can each be tapered in a direction away from the main portion of the body 902, which may further help guide the tips in position relative to each other and to the coupling mechanism 900.

The interior surface of the body 902 of the coupling mechanism 900 that defines the passages 904, 906, e.g., the interior surfaces of the extensions 910, 912, can include a gripping feature configured to facilitate secure seating of its associated drug delivery device therein. The gripping feature can have any of a variety of configurations, such as a textured surface, a rubber lining on the interior surface of the body 902, etc.

Each extension 910, 912 includes a depth guide 910d, 912d extending from a proximal end thereof. The depth guides 910d, 912d each include a pair of opposed flanges but can have another configuration, e.g., a single circumferential flange, more than two flanges arranged equidistantly around the extension 910, 912, etc. FIG. 13 shows the drug delivery device 200 in the process of being coupled to the coupling mechanism 900 such that the opening 210 of the drug delivery device's tip 208 is not yet in position proximal to the depth guide 910d of the first extension 910.

The body 902 in this illustrated embodiment is arced and thus has an arcuate shape. Longitudinal axes 910A, 912A of the extensions 910, 912 are thus not perpendicular to one another. Instead, the longitudinal axes 910A, 912A of the extensions 910, 912 intersect one another at a location distal to the body 902, e.g., on a side of the body 902 that is opposite to the extensions 910, 912. The arcuate shape of the body 902 thus prevents two drug delivery devices being coupled to the coupling mechanism 900 at the same time because there is insufficient space for the drug delivery devices distal to the body 902. The arcuate shape of the body 902 allows for the depth guides 910d, 912d to facilitate positioning of the drug delivery devices sequentially coupled to the coupling mechanism 900 to position the openings of the drug delivery devices at an optimal location within the user's nostril for optimum drug absorption in the correct region of the nasal cavity.

The coupling mechanism 900 is configured to be releasably coupled to the drug delivery devices 200 by being slid over each of the tips 208 similar to that discussed above regarding the coupling mechanism 600 of FIG. 9 and to be decoupled from the drug delivery devices 200 similar to that discussed above regarding the coupling mechanism 600 of FIG. 8.

As mentioned above, in some embodiments of the drug delivery device 100 of FIG. 1, the tip 112 includes two tips 112. FIGS. 14 and 15 illustrate such as an embodiment of the drug delivery device 100 of FIG. 1. The drug delivery device 1000 of FIGS. 14 and 15 is generally configured and used similar to the drug delivery device 200 of FIG. 2 and includes a handle portion 1012. However, in this illustrated embodiment, the drug delivery device 1000 includes two drug holders 1002, two pistons (obscured in FIGS. 14 and 15), two tips 1008, and two openings 1010. The drug holders 1002 are each configured to be pushed proximally relative to the handle portion 1012 to cause drug delivery, as discussed above. The drug holders 1002 can be pushed simultaneously such that drug sprays occur out of the openings 1010 simultaneously, or the drug holders 1002 can be pushed sequentially such that a drug spray occurs out of one of the openings 1010 before the other of the openings 1010.

The drug delivery device 1000 includes a depth guide 1004 that extends between the tips 1008. The depth guide 1004 is also configured to help stabilize the tips 1008 in a substantially fixed, perpendicular position relative to one another. A person skilled in the art will appreciate that the tips may not be precisely perpendicular to one another but nevertheless be considered to be substantially perpendicular to one another due to any number of factors, such as manufacturing tolerances and sensitivity of measurement equipment. The depth guide 1004 is located entirely between the tips 1008 in this illustrated embodiment but can additionally be alternatively located to the outer side of one or both of the tips 1008.

The drug delivery device 1000 includes first and second viewing windows 1006, 1016 formed in a side wall of each of the tips 1008. The first and second viewing windows 1006, 1016 are configured to be clearly visible to a user of the drug delivery device 1000 when the drug delivery device 1000 is held by hand. The viewing windows 1006, 1016 are circular in this illustrated embodiment but can have another shape, e.g., square, triangular, ovular, etc. The drug delivery device 1000 includes only two viewing windows 1006, 1016 in this illustrated embodiment but can have another number of viewing windows. For example, third and fourth viewing windows can be formed in a side wall of each of the tips 1008 substantially 180° around a circumference of each tip 1008 that are configured and used similar to the first and second viewing windows 1006, 1016. The third and fourth viewing windows may facilitate a user's visualization of at least one pair of viewing windows, e.g., the first and second viewing windows 1006, 1016 or the third and fourth viewing windows, regardless of whether the user's right or left hand is holding the drug delivery device 1000.

The viewing windows 1006, 1016 are configured to cooperate with a device indicator of the drug delivery device 1000. The device indicator is configured to indicate to a user whether a first dose has been dispensed, e.g., whether the first stage of operation has occurred, and whether a second dose has been dispensed, e.g., whether the second stage of operation has occurred. The device indicator is configured to be visible through the viewing windows 1006, 1016 to indicate whether the first and second doses have been dispensed. In this way, the user can know a drug delivery status of the drug delivery device 1000, including whether or not one or both of the first and second doses have been dispensed. Each of the drug holders 1002 in this illustrated embodiment has an amount of drug disposed therein for one spray, so indicating that a spray has been delivered thus indicates that substantially no drug remains in the drug holder 1002. Some drugs, such as esketamine, ketamine, and other controlled substances, may be required to have drug delivery therefrom verified per the drug's Risk Evaluation and Mitigation Strategies (REMS) to help, e.g., ensure that substantially no drug remains in the drug delivery device so that the drug deliverable therefrom is not accessed by an unauthorized party. A person skilled in the art will appreciate that the drug holder may not have precisely zero drug therein but nevertheless be considered to have substantially no drug therein due to any number of factors, such as sensitivity of measurement equipment.

The device indicator can have a variety of configurations. In this illustrated embodiment, the device indicator includes an area of each drug holder 1002. A first portion of each drug holder 1002 is a first color, and a second area of the drug holder 1002 is a second, different color. FIGS. 14 and 15 shows the first color visible through each of the viewing windows 1006, 1016. The first color being visible in a viewing window 1006, 1016 can indicate that the drug spray from the drug holder 1002 associated with the viewing window 1006, 1016 has not occurred. The second color being visible in a viewing window 1006, 1016 can indicate that the dose associated with the viewing window 1006, 1016 has occurred. Each of the drug holders 1002 moves proximally relative to the handle portion 1012, and thus relative to its associated viewing window 1006, 1016 during drug delivery, so the color visible through a viewing window 1006, 1016 can change from before the start of drug delivery, e.g., first color visible, to after the end of drug delivery, e.g., second color visible. The drug holders 1002 can each have the first color thereon in a first area and the second color thereon in a second area to allow the first and second colors to be sequentially visible through their respective viewing windows 1006, 1016. FIG. 16 illustrates the drug delivery device 1000 after one drug delivery has occurred such that the second color is visible in the first viewing window 1006 and the first color is visible in the second viewing window 1016. FIG. 17 illustrates the drug delivery device 1000 after two drug deliveries have occurred such that the second color is visible in the first and second viewing windows 1006, 1016. In other embodiments, the drug holders 1002 can have instead of or in addition to different colors another marking configured to be visible through the viewing windows 1006, 1016 to indicate whether the first and second doses have been dispensed, such as symbols (e.g., stars, triangles, "X"s, a thumbs up, a check mark, etc.), numbers (e.g., "0" for no spray occurred and "1" for spray occurred, and/or letters (e.g., "N" for no spray occurred and "Y" for spray occurred).

FIGS. 18-20 illustrate another embodiment of the drug delivery device 100 of FIG. 1 where the tip 112 includes two tips 112. The drug delivery device 1100 of FIGS. 18-20 is generally configured and used similar to the drug delivery device 1000 of FIGS. 14-17 except that instead of two viewing windows 1006, 1016, the drug delivery device 1100 includes a single window 1106. The first and second colored areas on the drug delivery device's drug holders 1102 are viewable in the viewing window 1106 similar to that discussed above regarding the drug delivery device 1000 of FIGS. 14-17. FIG. 18 illustrates the drug delivery device 1100 after no drug deliveries have occurred such that the first color is visible in the viewing window 1106 for each of the drug holders 1102 and tips 1108. FIG. 19 illustrates the drug delivery device 1100 after one drug delivery has occurred such that the second color is visible in the viewing window 1106 for the first drug holder 1102 and first tip 1108 and the first color is visible in the viewing window 1006 for the second drug holder 1102 and second tip 1108. FIG. 20 illustrates the drug delivery device 1100 after two drug deliveries have occurred such that the second color is visible in the viewing window 1106 for each of the drug holders 1102 and tips 1108. As discussed above with respect to the viewing windows 1006, 1016, the device indicator can have other configurations.

FIGS. 21-23 illustrate another embodiment of the drug delivery device 100 of FIG. 1 where the tip 112 includes two tips 112. The drug delivery device 1200 of FIGS. 21-23 is generally configured and used similar to the drug delivery device 1000 of FIGS. 14-17 except that instead of the two drug sprays being configured to be separately actuated, e.g., each drug holder 1002 being configured to be pushed independent of the other, the drug delivery device 1200 is configured such that the two drug sprays are configured to be actuated together such that the drug sprays out of the tips 1208 occur together. The drug delivery device 1200 thus includes a single actuator 1210 configured to be pushed proximally to cause the drug delivery device's two drug holders (obscured in FIGS. 21-23) to move proximally and thereby cause drug delivery, as discussed above. FIGS. 21 and 22 illustrate the drug delivery device 1200 before the start of drug delivery, e.g., before actuation of the actuator 1210, such that the first color is visible in the drug delivery device's first viewing window 1206 and in the drug delivery device's second viewing window 1216. FIG. 23 illustrates the drug delivery device 1200 after both drug sprays have been delivered, e.g., after actuation of the actuator 1210, such that the second color is visible in the first and second viewing windows 1106, 1216. As discussed above, the device indicator can have other configurations and/or the two viewing windows can instead be one viewing window.

The depth guide in the embodiments of FIGS. 14-20 has a vertical configuration between the drug delivery device's two tips. In this illustrated embodiment, the drug delivery device's depth guide 1204 has a horizontal configuration between the drug delivery device's two tips 1208.

The drug delivery device 1200 in this illustrated configuration is configured to move between an unlocked state, in which the actuator 1210 is allowed to be actuated, and a locked state, in which the actuator 1210 is prevented from being actuated. The drug delivery device 1200 being in the unlocked state versus the locked state also provides a visual indication in addition to the viewing windows 1206, 1216 as to whether or not drug delivery has occurred. FIGS. 21 and 22 illustrate the drug delivery device 1200 in the unlocked state. FIG. 23 illustrates the drug delivery device 1200 in the locked state.

The drug delivery device 1200 includes a locking feature 1212 and a locking mechanism 1214 configured to mate with the locking feature 1212. In the locked state of the drug delivery device 1200, the locking feature 1212 and the locking mechanism 1214 are mated to one another such that the actuator 1210 is prevented from being actuated. In the unlocked state of the drug delivery device 1200, the locking feature 1212 and the locking mechanism 1214 are not mated to one another such that the actuator 1210 is allowed to be actuated to deliver drug therefrom. The locking feature 1212 and the locking mechanism 1214 can have a variety of configurations. For example, as in this illustrated embodiment, the locking feature 1212 can include a hole, and the locking mechanism 1214 can include a protrusion configured to be seated in the hole. The protrusion 1214 is configured to be automatically seated in the hole 1212 in response to the actuation of the actuator 1210. The protrusion 1214 can be configured to flex in a distal direction such that when the actuator 1210 is moving proximally relative to the handle portion 1202, the protrusion 1214 can flex distally as the protrusion 1214 slides along an inner surface of the handle portion 1202 before encountering the hole 1212, into which the protrusion 1214 can then pop. The hole is formed in the drug delivery device's handle portion 1202 and the protrusion extends from the actuator 1210 in this illustrated embodiment, but instead the hole can be formed in the actuator 1210 and the protrusion can extend from the handle portion. In other embodiments, the locking feature 1212 can include a plurality of holes, and the locking mechanism 1214 can include a plurality of protrusions each configured to be seated in one of the holes. The hole 1212 and the protrusion 1214 have complementary rectangular shapes in this illustrated embodiment but can have another complementary shape, e.g., circular, ovular, etc. The hole 1212 and the protrusion 1214 can have different shapes from one another provided that the protrusion 1214 is still configured to be seated in the hole 1212, e.g., the hole 1212 being rectangular and the protrusion 1214 being a triangle with a point pointing radially outward, the hole being a square and the protrusion being a rectangle, etc.

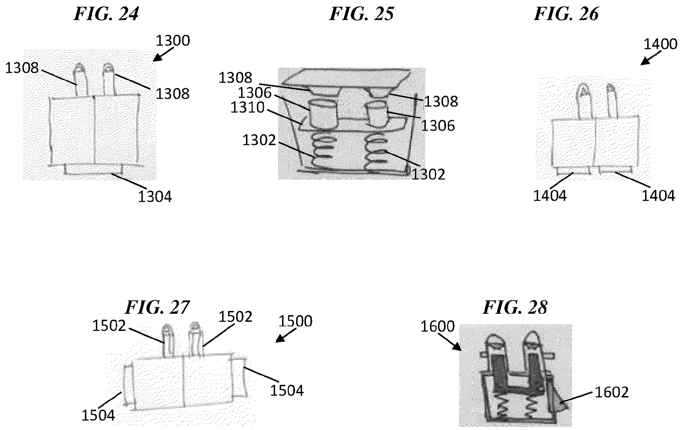

FIGS. 24 and 25 illustrate another embodiment of the drug delivery device 100 of FIG. 1 where the tip 112 includes two tips 112. The drug delivery device 1300 of FIGS. 24 and 25 is generally configured and used similar to the drug delivery device 1200 of FIGS. 21-23 except that the drug delivery device 1300 includes a spring 1302 that is operatively coupled to the drug delivery device's actuator 1304. The drug delivery device 1300 includes two springs 1302 but can include another number. The actuation of the actuator 1304, e.g., a user or other element pushing proximally on the actuator 1304, causes the spring 1302 to move from a compressed configuration, as in FIGS. 24 and 25, to an expanded configuration. The drug delivery device's drug holders 1306 are seated on a platform 1310 to which the spring 1302 is operatively coupled, e.g., by a proximal end of the spring 1302 being attached to a distal surface of the platform 1310. The actuation of the actuator 1304 causes the spring to expand and thereby push the platform 1306, and thus the drug holders 1306 seated thereon, in a proximal direction such that a piercing element 1308 pierces each of the drug holders 1306 and drug is delivered out of the drug delivery device's two tips 1308. The actuator 1304 and the platform 1310 can be mechanically coupled together before drug delivery, e.g., before actuation of the actuator 1306, which can hold the spring 1302 in the compressed configuration. Actuating the actuator 1304 can release the mechanical coupling of the actuator 1304 and the platform 1310 to allow the spring 1302 to expand and push the platform 1310 proximally. The mechanical coupling can have any of a variety of configurations, such as a rod attached to the actuator 1304 that pivots laterally to no longer be engaged with a distal surface of the platform 1306 in response to the actuator 1304 being pushed proximally, a protrusion that holds the platform 1310 in a fixed position relative to the actuator 1304 and that is configured to be retracted in response to the actuation of the actuator 1304 so as to no longer hold the platform 1310 in position, etc.

FIG. 26 illustrates another embodiment of the drug delivery device 100 of FIG. 1 where the tip 112 includes two tips 112. The drug delivery device 1400 of FIG. 26 is generally configured and used similar to the drug delivery device 1300 of FIGS. 24 and 25 except that the drug delivery device 1400 includes two actuators 1404. The actuators 1404 are each generally configured and used similar to the actuator 1304 of the drug delivery device 1300 except that the actuators 1404 of this illustrated embodiment are configured to be separately actuated to allow for the drug delivery device's to be delivered simultaneously, e.g., if the actuators 1404 are actuated simultaneously, or sequentially, e.g., if the actuators 1404 are actuated sequentially.

FIG. 27 illustrates another embodiment of the drug delivery device 100 of FIG. 1 where the tip 112 includes two tips 112. The drug delivery device 1500 of FIG. 27 is generally configured and used similar to the drug delivery device 1400 of FIG. 26 except that the drug delivery device 1500 includes two actuators 1504 that are located on sides of the drug delivery device 1500 instead of on a bottom of the drug delivery device 1500 opposite the drug delivery device's tips 1502 at a top of the drug delivery device 1500.

FIG. 28 illustrates another embodiment of the drug delivery device 100 of FIG. 1 where the tip 112 includes two tips 112. The drug delivery device 1600 of FIG. 28 is generally configured and used similar to the drug delivery device 1500 of FIG. 27 except that the drug delivery device 1500 includes a single actuator 1502 that is located on one side of the drug delivery device 1500 instead of two actuators on opposed sides of the drug delivery device 1600.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the spirit and scope of the claims may be made without departing from the overall scope of the present disclosure.

### Embodiments:

1. A drug delivery system, comprising:
   a first nasal drug delivery device comprising:
      a first tip configured to be positioned in a nose of a patient, the first tip having a first opening therein,
      a first drug holder containing a first drug therein, and
      a first actuator configured to be actuated and thereby cause the first drug to be delivered out of the first opening;
   a second nasal drug delivery device comprising:
      a second tip configured to be positioned in the nose of the patient, the second tip having a second opening therein,
      a second drug holder containing a second drug therein, and
      a second actuator configured to be actuated and thereby cause the second drug to be delivered out of the second opening; and
   a coupling mechanism comprising a body that defines first and second openings, the first opening being configured to releasably seat the first tip therein, and the second opening being configured to releasably seat the second tip therein.
2. The system of embodiment 1, wherein the first and second openings are located at respective proximal ends of the first and second drug delivery devices; and
   the coupling mechanism is configured such that the first tip seated in the first opening faces proximally and the second tip seated in the second opening faces proximally.
3. The system of embodiment 2, wherein the first and second actuators are configured to be selectively actuated so as to be actuated simultaneously to deliver the first and second drugs simultaneously into the nose or to be actuated sequentially to deliver the first and second drugs sequentially into the nose.
4. The system of embodiment 1, wherein the first and second openings are located at respective proximal ends of the first and second drug delivery devices; and
   the coupling mechanism is configured such that the first tip seated in the first opening faces proximally and the second tip seated in the second opening faces distally.
5. The system of embodiment 4, wherein the first and second actuators are configured to be actuated sequentially to deliver the first and second drugs sequentially into the nose.
6. The system of embodiment 1, wherein the first and second openings are located at respective proximal ends of the first and second drug delivery devices; and
   a proximal surface of the coupling mechanism defines a depth guide configured to contact a bottom surface of the nose with the coupling mechanism coupled to both of the first and second drug delivery devices and each of the first and second tips positioned in the nose.
7. The system of embodiment 1, wherein the coupling mechanism includes a release mechanism configured to move between a closed state, in which the body defines an enclosed perimeter that defines an enclosed perimeter of each of the first and second openings, and an open state, in which the body has a discontinuous perimeter and neither of the first and second openings have an enclosed perimeter.
8. The system of embodiment 1, wherein the first and second openings each have C-shapes that are back-to-back with one another.
9. The system of embodiment 1, wherein the first and second drug are each one of ketamine, esketamine, naloxone, and sumatriptan.
10. A drug delivery method, comprising:
   actuating each of the first and second actuators of any of embodiments 1-8 and thereby causing the first drug to be delivered out of the first opening and causing the second drug to be delivered out of the second opening.
11. The method of embodiment 10, wherein the first and second drugs are each one of ketamine, esketamine, naloxone, and sumatriptan.
12. A drug product disposed in the device of embodiment 1,
   wherein the drug product includes first and second drug products that each include one of ketamine, esketamine, naloxone, and sumatriptan.
13. A drug delivery device, comprising:
   a first tip configured to be positioned in a first nostril of a patient, the first tip having a first opening therein;
   a first drug holder containing a first drug therein configured to be delivered out of the first opening;
   a second tip configured to be positioned in a second nostril of the patient such that the first tip is positioned in the first nostril and the second tip is positioned in the second nostril, the second tip having a second opening therein;
   a second drug holder containing a second drug therein configured to be delivered out of the second opening; and
   a viewing window configured to indicate whether the first drug has or has not been delivered out of the first opening and whether the second drug has or has not bene delivered out of the second opening.
14. The device of embodiment 13, further comprising a first actuator configured to be actuated and thereby cause the first drug to be delivered out of the first opening; and
   a second actuator configured to be actuated and thereby cause the second drug to be delivered out of the second opening;
   wherein the actuation of the first actuator automatically causes the viewing window to indicate that the first drug has been delivered, and the actuation of the second actuator automatically causes the viewing window to indicate that the second drug has been delivered.
15. The device of embodiment 14, further comprising a first spring configured to be released in response to the actuation of the first actuator, the release of the first spring providing a force that moves the first drug holder relative to the first tip and thereby causes delivery of the first drug; and
   a second spring configured to be released in response to the actuation of the second actuator, the release of the second spring providing a force that moves the second drug holder relative to the second tip and thereby causes delivery of the second drug.
16. The device of embodiment 13, further comprising a single actuator configured to be actuated and thereby cause the first and second drugs to be delivered out of the first and second openings, respectively;
   wherein the actuation of the actuator automatically causes the viewing window to indicate that the first and second drugs have been delivered.
17. The device of embodiment 16, further comprising a locking mechanism configured to automatically lock the actuator in position relative to the first and second tips in response to the actuation of the actuator.
18. The device of embodiment 16, further comprising a spring configured to be released in response to the actuation of the actuator, the release of the spring providing a force that moves the first and second drug holders relative to the first and second tips and thereby causes delivery of the first and second drugs.
19. The device of embodiment 13, wherein a dispensing head of the drug delivery device has the first and second tips integrally formed therewith.
20. The device of embodiment 13, wherein the first drug holder includes a first portion that is a first color, and includes a second portion that is a second color;
   the second drug holder includes a first portion that is the first color, and includes a second portion that is the second color;
   the first color being visible in the viewing window is indicative that drug delivery has not occurred; and
   the second color being visible in the viewing window is indicative that drug delivery has occurred.
21. The device of embodiment 13, wherein the first and second drugs are each one of ketamine, esketamine, naloxone, and sumatriptan.
22. A drug delivery method, comprising:
   causing each of the first and second drugs of any of embodiments 13-20 to be delivered out of the first and second openings, respectively.
23. The method of embodiment 22, wherein the first and second drugs are each one of ketamine, esketamine, naloxone, and sumatriptan.
24. A drug product disposed in the device of embodiment 13,
   wherein the drug product includes first and second drug products that each include one of ketamine, esketamine, naloxone, and sumatriptan.

## Claims

1. A drug delivery device, comprising:
a first tip configured to be positioned in a first nostril of a patient, the first tip having a first opening therein;
a first drug holder containing a first drug therein configured to be delivered out of the first opening;
a second tip configured to be positioned in a second nostril of the patient such that the first tip is positioned in the first nostril and the second tip is positioned in the second nostril, the second tip having a second opening therein;
a second drug holder containing a second drug therein configured to be delivered out of the second opening; and
a viewing window configured to indicate whether the first drug has or has not been delivered out of the first opening and whether the second drug has or has not bene delivered out of the second opening.

2. The device of claim 1, further comprising a first actuator configured to be actuated and thereby cause the first drug to be delivered out of the first opening; and
a second actuator configured to be actuated and thereby cause the second drug to be delivered out of the second opening;
wherein the actuation of the first actuator automatically causes the viewing window to indicate that the first drug has been delivered, and the actuation of the second actuator automatically causes the viewing window to indicate that the second drug has been delivered.

3. The device of claim 2, further comprising a first spring configured to be released in response to the actuation of the first actuator, the release of the first spring providing a force that moves the first drug holder relative to the first tip and thereby causes delivery of the first drug; and
a second spring configured to be released in response to the actuation of the second actuator, the release of the second spring providing a force that moves the second drug holder relative to the second tip and thereby causes delivery of the second drug.

4. The device of claim 1, further comprising a single actuator configured to be actuated and thereby cause the first and second drugs to be delivered out of the first and second openings, respectively;
wherein the actuation of the actuator automatically causes the viewing window to indicate that the first and second drugs have been delivered.

5. The device of claim 4, further comprising a locking mechanism configured to automatically lock the actuator in position relative to the first and second tips in response to the actuation of the actuator.

6. The device of claim 4, further comprising a spring configured to be released in response to the actuation of the actuator, the release of the spring providing a force that moves the first and second drug holders relative to the first and second tips and thereby causes delivery of the first and second drugs.

7. The device of claim 1, wherein a dispensing head of the drug delivery device has the first and second tips integrally formed therewith.

8. The device of claim 1, wherein the first drug holder includes a first portion that is a first color, and includes a second portion that is a second color;
the second drug holder includes a first portion that is the first color, and includes a second portion that is the second color;
the first color being visible in the viewing window is indicative that drug delivery has not occurred; and
the second color being visible in the viewing window is indicative that drug delivery has occurred.

9. The device of claim 1, wherein the first and second drugs are each one of ketamine, esketamine, naloxone, and sumatriptan.
